# NOUVEAU FASCICULE DE BREVET EUROPEEN

(11) **EP 0 925 340 B2**
(45) Date de publication et mention de la décision concernant l'opposition: **20.09.2006**
(45) Mention de la délivrance du brevet: 16.04.2003
(21) Numéro de dépôt: 97927153.3
(22) Date de dépôt: 04.06.1997
(51) Int. Cl.: C11B 1/00, C11B 5/00, A23D 9/06, A23L 1/39

(54) **EXTRACTION D'ANTIOXIDANTS**
ENTFERNUNG VON ANTIOXIDANTIEN
REMOVAL OF ANTIOXIDANTS

(30) Priorité: 08.06.1996 EP 96201590
(43) Date de publication de la demande: 30.06.1999
(73) Titulaire: SOCIETE DES PRODUITS NESTLE S.A., 1800 Vevey (CH)
(72) Inventeur: BRACCO, Umberto, CH-1800 Vevey (CH); AESCHBACH, Robert, CH-1800 Vevey (CH); ROSSI, Patricia, CH-1814 La Tour-de-Peilz (CH)
(74) Mandataire: Thomas, Alain
(86) Numéro de dépôt international: PCT/EP1997/002967
(87) Numéro de publication internationale: WO 1997/047711

(56) Documents cités:
- EP-A- 0 639 336
- EP-A- 0 686 353
- WO-A-93/17567
- SU-A- 956 552
- DATABASE WPI Week 8939 Derwent Publications Ltd., London, GB; AN 89-280269 XP002018838 & ES 2 005 480 A (JIMENEZ GARRIDO A) , 1 mars 1989
- DATABASE WPI Week 8639 Derwent Publications Ltd., London, GB; AN 86-257415 XP002018839 & SU 1 211 280 A (PETROCHEM PROCESS) , 15 février 1986
- PATENT ABSTRACTS OF JAPAN vol. 96, no. 9, 30 septembre 1996 & JP 08 119825 A (ICHIMARU PHARCOS CO LTD), 14 mai 1996,
- DATABASE WPI Week 8436 Derwent Publications Ltd., London, GB; AN 84-224461 XP002018840 & SU 1 066 603 A (AS AZERB PETROCHEM) , 15 janvier 1984
- M. BRENES ET AL.: "Biochemical changes in phenolic compounds during Spanish-style green olive processing" JOURNAL OF AGRICULTURAL AND FOOD CHEMISTRY, vol. 43, no. 10, 1995, WASHINGTON US, pages 2702-2706, XP002041724
- R. AESCHBACH ET AL.: "Antioxidant actions of thymol, carvacrol, 6-gingerol, zingerone and hydroxytyrosol" FOOD AND CHEMICAL TOXICOLOGY, vol. 32, no. 1, 1994, GB, pages 31-36, XP000609887
- A. VAZQUEZ RONCERO: "Les polyphénols de l'huile d'olive et leur influence sur les caractéristiques de l'huile" REVUE FRANCAISE DES CORPS GRAS, vol. 25, no. 1, 1978, PARIS FR, pages 21-26, XP002018837
- GIORGIO BIANCHI ET AL.: "3,4-Dihydroxyphenylglycol, a major C6-C2 phenolic in Olea europaea fruits" PHYTOCHEMISTRY, vol. 35, no. 5, 1994, GB, pages 1335-1337, XP000609833

## Description

La présente invention a pour objet un procédé d'extraction d'antioxydants d'olive, une fraction lipidique enrichie en antioxydants, un extrait enrichi en antioxydants ainsi qu'une composition alimentaire ou cosmétique, contenant cette fraction lipidique et/ou cet extrait.

Dans un procédé classique, les olives sont traitées par pression et l'on obtient trois phases, une phase aqueuse, une phase lipidique et une phase solide. Dans un tel procédé, les antioxydants hydrosolubles sont perdus dans la phase aqueuse et sont tellement dilués dans la phase aqueuse, que l'on ne peut plus les récupérer.
De plus, la phase aqueuse, constituant les eaux usées dont le volume est approximativement quatre fois supérieure au volume de la phase lipidique, doit être traitée en station d'épuration.

Ainsi, A. Uzzan (Manuel des corps gras - ISBN 2 - 85206 - 662/9 - 1992 - 763-768) décrit notamment un procédé d'obtention de l'huile d'olive par pressage, dans lequel les olives sont nettoyées, malaxées puis passées dans une presse hydraulique, de manière à séparer la phase liquide et la phase solide. A ce stade, la phase liquide est divisée par décantation ou par centrifugation en ses deux constituants, la phase aqueuse contenant les substances hydrosolubles de l'olive et l'huile d'olive. Ces deux constituants sont encore une fois centrifugés, de manière, d'une part, à recueillir l'huile clarifiée et purifiées et, d'autre part, à extraire l'huile résiduelle contenue dans la phase aqueuse. Cette phase aqueuse ainsi que la phase solide précédente, encore riches en antioxydants, sont éliminées.

SU-A-1211280 décrit un procédé de fabrication d'huile d'olives, dans lequel on congèle les olives à une température de - 70° C à - 190° C, on sépare la pulpe des noyaux, on sèche la pulpe à 50-60° C et l'on effectue ensuite une extraction de l'huile d'olives en présence d'un solvant non polaire, ce qui conduit à une fraction contenant les antioxydants liposolubles d'olive, tels que le tocophérol.

JP-A- 08119825 décrit un produit cosmétique à base d'hydroxytyrosol en tant que composant actif, ce composant pouvant être extrait d'huile d'olive ou obtenu par synthèse.

EP-A-0896612 divulgue une huile d'olive à laquelle on ajoute une dispersion d' antioxydants d'origine organique et minérale dans le glycérol, constituant un complexe d'antioxydants et de vitamines, lesquels antioxydants n'étant pas naturellement présents dans l'huile d'olive, c'est à dire provenant de feuilles ou pépins de plantes autres que l'olivier.

Le problème à la base de l'invention est la mise à disposition d'une palette d'antioxydants d'olive plus large que celle qui pouvait être obtenue par mise en oeuvre des procédés d'extraction et d'enrichissement connus, par un procédé d'extraction à sec simple et écologique n'impliquant pas la perte des antioxydants hydrosolubles.

La présente invention a pour but de proposer un procédé permettant de recueillir, à partir d'olives vertes eV ou d'olives plus ou moins mûres, d'une part une fraction lipidique enrichie en antioxydants et d'autre part un extrait enrichi en antioxydants.

A cet effet, dans le procédé d'extraction d'antioxydants d'olive selon la présente invention:
- on broie des olives,
- on sèche sous vide ces olives broyées, de manière à obtenir des olives séchées riches en antioxydants hydrosolubles,
- on effectue un pressage de ces olives séchées, de manière à recueillir une fraction lipidique enrichie en antioxydants et un tourteau,
- on effectue sur le tourteau au moins une extraction à chaud au MCT, c'est à dire un mélange de triglycérides à chaîne moyenne, ou avec un alkylène glycol en C₂-C₆ par pression supérieure ou égale à 40 bar,
- puis l'on recueille un extrait enrichi en antioxydants à partir du tourteau.

On a constaté avec surprise qu'un tel procédé permet effectivement d'obtenir une fraction lipidique et un extrait enrichis en antioxydants et, plus particulièrement, en antioxydants hydrosolubles.
De plus, le procédé selon la présente invention, du fait de l'absence d'eaux usées, présente des avantages écologiques évidents.

On peut choisir les olives parmi les olives vertes et/ou les olives mûries, par exemple.

On peut congeler les olives, de manière à faciliter le broyage, par exemple.

On broie donc les olives. Pour ce faire, on peut utiliser des techniques usuelles pour le broyage des fruits à noyaux, notamment des moulins à marteaux, à disques, colloïdaux, à molasses ou un cutter à lames.

On peut traiter enzymatiquement les olives broyées, à l'aide d'enzymes d'origine bactérienne ou fongique, d'hydrolases, de glucosidases ou de polyphénolhydrolases, par exemple, de manière à hydrolyser les glycosides et améliorer l'extraction des antioxydants, par exemple.

On peut sécher sous vide les olives broyées à une température inférieure ou égale à 80° C, de manière à recueillir des olives séchées riches en antioxydants hydrosolubles et dont la teneur en eau est de 1-20% en poids, par exemple. De préférence, on sèche, de manière à obtenir des olives séchées dont la teneur en eau est de 5-10% en poids. On favorise, ainsi, uniquement la formation de deux phases, la fraction lipidique enrichie en antioxydants et le tourteau, lors de l'étape de pressage.

On peut sécher par lyophilisation à une pression réduite de 10⁻³-10⁻¹ bar ou dans une étuve à vide à une pression réduite de 0,1-0,2 bar, par exemple.

On peut préchauffer les olives séchées et les maintenir à chaud pendant une certaine durée, avant d'effectuer le pressage, de manière à augmenter la teneur en antioxydants de la fraction lipidique.

On effectue donc un pressage sur ces olives séchées, de manière à recueillir une fraction lipidique enrichie en antioxydants et un tourteau. On peut effectuer ce pressage à température ambiante ou à chaud dans une presse à piston munie d'une cage filtrante, notamment une presse de type Carver commercialisée par la société Fred S. Carver, Menomonee Falls, Wisconsin - USA, de manière à presser et à filtrer en une seule étape.

Puis, on effectue donc sur le tourteau au moins une extraction à chaud au MCT ou avec un alkylène glycol en C₂-C₆ par pression supérieure ou égale à 40 bar. On peut effectuer sur le tourteau au moins une extraction dans un rapport pondéral MCT ou alkylène glycol en C₂-C₆/ tourteau de 0,5 à 2, par exemple. On peut effectuer cette ou ces extractions à chaud dans une presse à piston munie d'une cage filtrante, de type Carver.
L'alkylène glycol peut être le glycol, le 1,2-propylène glycol ou le 1,3-butylène glycol, par exemple.
Lors d'une extraction à chaud au MCT par pression, on isole principalement les antioxydants liposolubles et lors d'une extraction à chaud avec un alkylène glycol en C₂-C₆ par pression, on isole les antioxydants liposolubles et les antioxydants hydrosolubles.

La présente invention concerne également une fraction lipidique selon la revendication 8.

De plus, la présente invention concerne un extrait selon la revendication 9.

Cet extrait comprend des antioxydants hydrosolubles, notamment l'hydroxytyrosol, le tyrosol, des acides phénoliques et l'oleuropéine.

La présente invention concerne une composition alimentaire ou cosmétique selon la revendication 10.

De plus, la présente invention concerne une composition alimentaire ou cosmétique selon la revendication 11.

Le procédé selon la présente invention est décrit plus en détails dans les exemples non limitatifs ci-après.
Dans ces exemples, les pourcentages sont donnés en poids, sauf indication contraire.

### Exemple 1

On broie à l'aide du broyeurAlpina, commercialisé par la société C. Hoegger et Cie AG, Gossau, CH- St Gall, 1 kg d'olives vertes congelées dont la teneur globale en eau est de 55% en poids.

On sèche ces olives vertes broyées dans une étuve sous vide de type Inox Maurer 20, commercialisée par la société Inox Maurer AG, Trimbach, CH - Soleure, à une température de 55° C et à une pression réduite de 0,1 bar, de manière à obtenir des olives vertes séchées dont la teneur globale en eau est de 6% en poids.

Puis, l'on effectue un pressage à température ambiante sur ces olives vertes séchées dans une presse à piston, de type Carver, à 500 bar pendant 60 min, de manière à recueillir la fraction lipidique enrichie en antioxydants et le tourteau.

Puis, l'on effectue sur 50 g du tourteau ainsi recueilli une extraction à chaud au 1,2 propylène glycol.
Pour ce faire, aux 50 g de tourteau on ajoute 50 g de 1,2-propylène glycol. On laisse le tout sous agitation pendant 60 min à 80° C et l'on presse le mélange dans une presse à piston, de type Carver, à 500 bar pendant 60 min.

On recueille ainsi l'extrait enrichi en antioxydants. Le test Rancimat® à 110° C dans différentes graisses et huiles donne le pouvoir antioxydant sous forme d'indices antioxydants indiqués dans le tableau I ci-après. L'indice antioxydant est défini comme étant le rapport:

### Test d'oxydation Rancimat® à 110° C:

On place l'échantillon dans un réacteur fermé.
L'échantillon est chauffé à 110° C et est saturé en oxygène provenant de l'air introduit dans le réacteur. Durant l'oxydation, le réacteur est lui-même relié par un tube souple à un récipient contenant de l'eau distillée et où est plongée une électrode de platine.
Les composés volatiles entraînent une augmentation de la conductivité.
La conductivité est mesurée et les périodes d'induction sont calculées.
On détermine le temps d'induction graphiquement à partir de la courbe transcrite de la conductivité en fonction du temps par intersection de la tangente à la courbe avec l'axe des temps.

### Exemple comparatif 1

On procède de la manière telle que décrite à l'exemple 1, à l'exception du fait que l'on effectue sur 50 g du tourteau recueilli une extraction à chaud par voie organique, à l'éthanol à 85%.

Pour cefaire, aux 50 g de tourteau on ajoute 100 ml d'éthanol à 85%. On laisse le tout sous agitation pendant 60 min à 80° C et l'on filtre, avant de concentrer à 50% de volume. Puis, on ajoute 50 g de propylèneglycol, on évapore l'éthanol et l'on centrifuge pendant 10 min à 3000 rpm, pour clarifier.

On recueille un extrait contenant les antioxydants. Le test Rancimat® à 110° C dans différentes graisses et huiles donne le pouvoir antioxydant sous forme d'indices antioxydants indiqués dans le tableau I ci-après,

**Tableau I**

| Pour le test Rancimat ®, les mesures sont effectuées en présence de 2% d'extrait par rapport à la graisse de poule ou par rapport à l'huile d'olive. | | |
|---|---|---|
| | Test Rancimat ® /110° C (indice antioxydant) | |
| Exemples | huile d'olive | graisse de poule |
| 1 | 3,8 | 8,6 |
| i | 4,5 | 9,9 |

Les mesures du pouvoir antioxydant, indiquées sous forme d'indice antioxydant, au tableau I mettent en évidence la qualité et la stabilité oxydative de l'extrait obtenu par la mise en oeuvre par le procédé selon la présente invention (exemple 1). Cette qualité et cette stabilité oxydative sont comparables à celles que l'on a pour un extrait obtenu par la mise en oeuvre d'un procédé plus complexe, dans lequel on effectue une extraction à chaud par voie organique (exemple i).

### Exemple 2

On procède de la manière décrite à l'exemple 1, à l'exception du fait que l'on effectue les mesures pour le test Rancimat® en présence de 1 % d'extrait par rapport à la graisse de poule ou par rapport à l'huile d'olive.

Par ailleurs, on mesure le pouvoir antioxydant sous forme d'un indice antioxydant par la méthode d'électrode à oxygène à 30° C dans de l'huile de maïs. On effectue ces mesures en présence de 1 % d'extrait par rapport à l'huile de maïs.

De plus, on mesure par le test Rancimat® à 120° C, le temps d'induction de la fraction lipidique recueillie après le pressage à température ambiante des olives vertes séchées. La valeur du temps d'induction de la fraction lipidique est mentionnée au tableau III ci-après.

### Test d'oxydation: électrode à oxygène à 30° C.

On prépare une émulsion en mélangeant 5 % d'huile et le % indiqué en antioxydants par rapport à l'huile. dans une solution tampon de pH 7( No.9477, Merck, Darmstadt, D) avec 0,1 % d'émulsifiant par agitation vigoureuse sous azote pendant 30 min que l'on émulsifie par 6 passages consécutifs à 30° C dans un microfluidiseur H 5000.
On mesure ensuite la stabilité oxydative de l'émulsion à l'aide d'une électrode TRI OX EO 200® couplée avec un oxygène-mètre OXI 530®.
On attend 5 à 10 min jusqu'à ce que le pourcentage de saturation d'oxygène ait une valeur constante.
Cette mesure s'effectue à 30° C sous agitation en vase clos, après adjonction de 5 ml du catalyseur Hemin (Fluka AG, Buchs, CH) à 100 ml d'émulsion. Le catalyseur Hemin est préparé à partir de 52 mg d'Hemin dans 100 ml d'eau, auxquels on ajoute 8 gouttes de KOH à 10%.
Le temps d'induction représente la durée en heures pour une absorption totale de l'oxygène dissout.

Le test Rancimat® à 110° C dans différentes graisses et huiles et la méthode d'électrode à oxygène à 30° C dans l'huile de maïs donnent le pouvoir antioxydant sous forme d'indices antioxydants indiqués dans le tableau II ci-après.

### Exemple comparatif ii

On procède de la manière décrite à l'exemple 2, à l'exception du fait que l'on ne sèche pas les olives vertes broyées.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

La valeur du temps d'induction à 120° C de la fraction lipidique est mentionnée au tableau III ci-après.

### Exemple 3

On procède de la manière telle que décrite à l'exemple 1, à l'exception du fait que l'on préchauffe à 70° C pendant 60 min les olives séchées avant d'effectuer le pressage.

Après refroidissement à température ambiante, on effectue le pressage à température ambiante dans une presse à piston, de type Carver®, à 500 bar pendant 60 min. On recueille ainsi la fraction lipidique enrichie en antioxydants et le tourteau.

On mesure, par le test Rancimat® à 120° C, le temps d'induction de la fraction lipidique. La valeur du temps d'induction de la fraction lipidique est mentionnée au tableau III ci-après.

Par ailleurs, on effectue sur 50 g du tourteau recueilli une extraction à chaud au 1,2 propylène glycol.
Pour ce faire, aux 50 g de tourteau on ajoute 50 g de 1,2-propylène glycol. On laisse le tout sous agitation pendant 60 min à 80° C et l'on presse le mélange dans une presse à piston, de type Carver, à 500 bar pendant 60 min.

On recueille l'extrait enrichi en antioxydants.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

### Exemple 4

On procède de la manière décrite à l'exemple 2, à l'exception du fait que l'on effectue sur 50 g du tourteau recueilli une extraction à chaud au MCT.

Pour ce faire, aux 50 g de tourteau on ajoute 50 g de MCT. On laisse le tout sous agitation pendant 60 min à 80° C et l'on presse le mélange dans une presse à piston, de type Carver, à 500 bar pendant 60 min.

On recueille l'extrait enrichi en antioxydants.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

### Exemple comparatif iv

On procède de la manière décrite à l'exemple 4, à l'exception du fait que l'on ne sèche pas les olives vertes broyées.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

### Exemple 5

On procède de la manière décrite à l'exemple 4, à l'exception du fait que l'on préchauffe à 70° C pendant 60 min les olives vertes séchées avant d'effectuer le pressage.

Après refroidissement à température ambiante, on effectue le pressage à température ambiante dans une presse à piston, de type Carver®, à 500 bar pendant 60 min. On recueille ainsi la fraction lipidique enrichie en antioxydants et le tourteau.

On mesure par le test Rancimat® à 120° C, le temps d'induction à partir de 1% de fraction lipidique ainsi recueillie.

Par ailleurs, on effectue sur 50 g du tourteau recueilli une extraction à chaud au MCT.
Pour ce faire, aux 50 g de tourteau on ajoute 50 g de MCT. On laisse le tout sous agitation pendant 60 min à 80° C et l'on presse le mélange dans une presse à piston, de type Carver, à 500 bar pendant 60 min.

On recueille l'extrait enrichi en antioxydants.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau Il ci-après.

**Tableau II**

| On effectue toutes les mesures en présence de 1% d'extrait par rapport à l'huile de maïs, par rapport à la graisse de poule ou par rapport à l'huile d'olive. | | | |
|---|---|---|---|
| | Test Rancimat® / 110°C (indice antioxydant) | | Test électrode à oxygène / 30°C (indice antioxydant) |
| Exemples | graisse de poule | huile d'olive | huile de maïs |
| 2 | 4,3 | 2,1 | 7,8 |
| ii | 1,8 | 1,1 | 1,5 |
| 3 | 3,9 | 1,6 | 8,4 |
| 4 | 3,2 | 1,3 | - |
| iv | 1, 2 | 1 | - |
| 5 | 1,9 | 1,2 | - |

| | | | |
|---|---|---|---|
| -: non testé | | | |

Les mesures du pouvoir antioxydant, indiquées sous forme d'indice antioxydant, respectivement aux exemples 2 et il et aux exemples 4 et iv dans le tableau II, mettent en évidence une augmentation de la stabilité oxydative de l'extrait enrichi en antioxydants, obtenu à partir du tourteau, dans le cas où l'on sèche les olives vertes broyées.

De plus, les mesures du pouvoir antioxydant, indiquées sous forme d'indice antioxydant, respectivement aux exemples 2 et 3 et aux exemples 4 et 5 dans le tableau II, mettent en évidence le fait que si l'on effectue un pressage à chaud sur les olives vertes séchées, l'extrait enrichi en antioxydants, à partir du tourteau, présente une stabilité oxydative plus faible.

Enfin, les mesures du pouvoir antioxydant, indiquées sous forme d'indice antioxydant dans le test d'électrode à oxygène, aux exemples 2 et 3, mettent en évidence la qualité et la stabilité oxydative des composés antioxydants en émulsion, par rapport aux résultats obtenus dans une huile dans le test d'oxydation Rancimat®.

**Tableau III**

| Exemples | Test Rancimat®/ 120°C (temps d'induction en heures) |
|---|---|
| 2 | 25 |
| ii | 11 |
| 3 | 42 |

Les mesures du pouvoir antioxydant, indiquées sous forme de temps d'induction, dans le tableau III, mettent en évidence le fait que si l'on préchauffe les olives vertes séchées avant d'effectuer le pressage à température ambiante, la fraction lipidique enrichie en antioxydants présente une stabilité oxydative accrue.

### Exemple 6

On procède de la manière telle que décrite à l'exemple 2, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

### Exemple comparatif vi

On procède de la manière telle que décrite à l'exemple ii, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

### Exemple 7

On procède de la manière telle que décrite à l'exemple 4, à l'exception du fait que les olives sont des olives mûries,

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

### Exemple comparatif vii

On procède de la manière telle que décrite à l'exemple iv, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

**Tableau IV**

| On effectue toutes les mesures en présence de 1% d'extrait par rapport à l'huile de maïs ou par rapport à la graisse de poule. | | |
|---|---|---|
| | Test Rancimat ® / 110°C (indice antioxydant) | Test électrode à oxygène / 30°C (indice antioxydant) |
| Exemple | graisse de poule | huile de maïs |
| 6 | 1,8 | 1,9 |
| vi | 1,2 | 1,7 |
| 7 | 1,1 | 1,3 |
| vii | 1 | 1,3 |

Les mesures du pouvoir antioxydant, indiquées sous forme d'indice antioxydant, respectivement aux exemples 6 et vi et aux exemples 7 et vii dans le tableau IV, mettent en évidence une augmentation de la stabilité oxydative de l'extrait enrichi en antioxydants, obtenu à partir du tourteau, dans le cas où l'on sèche les olives mûries et préalablement broyées.

### Exemple 8

On réalise la stabilisation d'une huile d'olive non vierge du commerce (olio Sasso ®) avec un extrait enrichi en antioxydants d'olive préparé conformément à l'exemple 1.

A 100 g d'huile d'olive, on ajoute 2 g de l'extrait et on obtient un huile 3,8 fois plus protégée que l'huile sans l'extrait par mesure du pouvoir antioxydant sous forme d'indice antioxydant dans le test Rancimat ® à 110° C.

### Exemple 9

On prépare une sauce pesto contenant de l'extrait enrichi en antioxydants, tel qu'obtenu à l'exemple 4.

Pour ce faire, on prépare, sous agitation, à température ambiante, un mélange, pour la sauce pesto, contenant, par kg de sauce, 463,5 g d'un mélange d'hu ile d'olives et d'huile de tournesol, 165 g de basilic, 164 g de parmesan râpé, 73 g de poudre de petit lait, 72,5 g de persil, 45 g de pignons broyés, 7 g de sel, 8 g, d'ail lyophilisé et 2 g de poivre blanc moulu.

Puis, on ajoute, à ce mélange, 2% d'extrait enrichi en antioxydants, tout en agitant, de manière à répartir l'extrait de façon homogène dans la sauce pesto ainsi préparée.

On conditionne alors cette sauce pesto dans des barquettes en plastique de 100 g que l'on stocke ensuite à 4-7° C.

### Exemple 10

On prépare une sauce pesto contenant une fraction lipidique enrichie en antioxydants , telle qu'obtenue à l'exemple 3.

Pour ce faire, on prépare, sous agitation, à température ambiante, un mélange, pour la sauce pesto, contenant, par kg de sauce, 233,5 g de fraction lipidique enrichie en antioxydants, 230 g d'huile de tournesol, 165 g de basilic, 164 g de parmesan râpé, 72,5 g de poudre de petit lait, 45 g de pignons broyées, 7 g de sel, 8 g d'ail lyophilisé et 2 g de poivre blanc moulu.

Puis l'on conditionne alors cette sauce pesto dans des barquettes en plastique de 100 g que l'on stocke ensuite à 4-7° C.

### Exemple 11

On prépare une sauce aux tomates contenant unefraction lipidique enrichie en antioxydants , telle qu'obtenue à l'exemple 5.

Pour ce faire, on prépare, sous agitation à 50° C, un mélange, pour la sauce aux tomates, contenant, par kg de sauce, 302,4 g de purée de tomates, 30 g de fraction lipidique enrichie en antioxydants, 34,5 g d'amidon, 17 g de sucre, 2,5 g d'oignons lyophilisés, 0,9 g de poivre blanc moulu, 8,5 g de sel, 2,5 g d'origan, 0,4 g de basilic haché finement et 601,3 g d'eau.

Puis, on conditionne à température ambiante la sauce aux tomates ainsi préparée dans des boîtes de 150 g fermées hermétiquement.

### Exemple 12

On prépare une sauce aux tomates contenant de l'extrait enrichi en antioxydants, tel qu'obtenu à l'exemple 1.

Pour ce faire, on prépare, sous agitation à 50° C, un mélange, pour la sauce aux tomates, contenant, par kg de sauce, 302,4 g de purée de tomates, 30 g d'huile de tournesol, 34,5 g d'amidon, 17 g de sucre, 2,5 g d'oignons lyophilisés, 0,9 g de poivre blanc moulu, 8,5 g de sel, 2,5 g d'origan, 0,4 g de basilic haché finement et 601,3 g d'eau.

Puis, on ajoute, à ce mélange, 2% d'extrait enrichi en antioxydants, tout en agitant, de manière à répartir l'extrait de façon homogène dans la sauce aux tomates ainsi préparée.

On conditionne alors à température ambiante la sauce aux tomates ainsi préparée dans des boîtes de 150 g fermées hermétiquement.

## Revendications

1. Procédé d'extraction d'antioxydants d'olives, dans lequel:
- on broie des olives,
- on sèche sous vide ces olives broyées, de manière à obtenir des olives séchées riches en antioxydants hydrosolubles,
- on effectue un pressage de ces olives séchées, de manière à recueillir une fraction lipidique enrichie en antloxydants et un tourteau,
- on effectue sur le tourteau au moins une extraction à chaud au MCT ou avec un alkylène glycol en C₂-C₆ par pression supérieure ou égale à 40 bar,
- puis l'on recueille un extrait enrichi en antioxydants à partir du tourteau.

2. Procédé selon la revendication 1, dans lequel les olives sont des olives vertes et/ou des olives mûries.

3. Procédé selon la revendication 1, dans lequel les olives sont des olives congelées.

4. Procédé selon la revendication 1, dans lequel on sèche les olives broyées sous vide à une température inférieure ou égale à 80° C, de manière à obtenir des olives séchées riches en antioxydants hydrosolubles et dont la teneur globale en eau est de 1-20% en poids.

5. Procédé selon la revendication 1, dans lequel les olives séchées ont une teneur globale en eau de 5-10% en poids.

6. Procédé selon la revendication 1, dans lequel on effectue sur le tourteau au moins une extraction dans un rapport pondéral MCT ou alkylène glycol en C₂-C₆/tourteau de 0,5 à 2.

7. Procédé selon la revendication 1, dans lequel l'alkylène glycol en C₂-C₆ est le glycol, le 1,2-propylène glycol ou le 1,3-butylène glycol.

8. Fraction lipidique de pressage d'olives entières séchées, contenant des antioxydants liposolubles et hydrosolubles d'olive, dont le temps d'induction est 15-75 h à 110-120° C et qui est susceptible d'être obtenue par la mise en oeuvre du procédé selon la revendication 1.

9. Extrait contenant des antioxydants liposolubles et hydrosolubles d'olive comprenant l'hydroxytyrosol, le tyrosol, des acides phénoliques et l'oleuropéine, susceptible d'être obtenu par la mise en oeuvre du procédé selon l'une des revendications 1 à 7.

10. Composition alimentaire ou cosmétique sous forme de dispersion ou d'émulsion, comprenant 0,5-4% on poids d'extrait enrichi en antioxydants liposolubles et hydrosolubles d'olive selon là revendication 9.

11. Composition alimentaire ou cosmétique sous forme de dispersion ou d'émulsion, comprenant une fraction lipidique enrichie en antioxydants hydrosolubles d'olive selon la revendication 8

## Claims

1. Process for extracting olive antioxidants, in which:
- olives are comminuted,
- these comminuted olives are dried under vacuum so as to obtain dried olives rich in water-soluble antioxidants,
- these dried olives are pressed so as to collect an antioxidant-enriched lipid fraction and a cake,
- at least one hot extraction with MCT or with a C₂-C₆ alkylene glycol at a pressure of greater than or equal to 40 bar is performed on the cake,
- an antioxidant-enriched extract is then collected from the cake.

2. Process according to claim 1, in which the olives are green olives and/or ripened olives.

3. Process according to claim 1, in which the olives are frozen olives.

4. Process according to claim 1, in which the comminuted olives are dried under vacuum at a temperature of less than or equal to 80°C, so as to obtain dried olives rich in water-soluble antioxidants, the overall water content of which is 1-20% by weight.

5. Process according to claim 1, in which the dried olives have an overall water content of 5-10% by weight.

6. Process according to claim 1, in which at least one extraction with an MCT or C₂-C₆ alkylene glycol/cake weight ratio of 0.5 to 2 is performed on the cake.

7. Process according to claim 1, in which the C₂-C₆ alkylene glycol is glycol, 1,2-propylene glycol or 1,3-butylene glycol.

8. Lipid fraction from the pressing of dried whole olives, containing liposoluble and water-soluble olive antioxidants, the induction time of which is 15-75 hours at 110-120°C, and which may be obtained by carrying out the process according to claim 1.

9. Extract containing liposoluble and water-soluble olive antioxidants comprising hydroxytyrosol, tyrosol, phenolic acids and oleuropein, which may be obtained by carrying out the process according to one of claims 1 to 7.

10. Cosmetic or food composition in the form of a dispersion or an emulsion, comprising 0.5-4 wt.% of extract enriched with liposoluble and water-soluble olive antioxidants according to claim 9.

11. Cosmetic or food composition in the form of a dispersion or emulsion, comprising a lipid fraction enriched with hydrosoluble olive antioxidants according to claim 8.

## Patentansprüche

1. Verfahren zur Extraktion von Antioxidantien aus Oliven, bei dem man
- Oliven zerkleinert,
- die zerkleinerten Oliven unter Unterdruck so trocknet, dass man getrocknete Oliven mit einem hohen Gehalt an wasserlöslichen Antioxidantien erhält,
- eine Pressung dieser getrockneten Oliven so vornimmt, dass man eine mit Antioxidantien angereicherte Lipidfraktion und einen Kuchen erhält,
- an dem Kuchen mindestens eine Extraktion in der Wärme mit MCT oder mit einem C₂-C₆-Alkylenglykol durch Druck über oder gleich 40 bar vornimmt,
- dann aus dem Kuchen einen mit Antioxidantien angereicherten Extrakt gewinnt.

2. Verfahren nach Anspruch 1, bei dem die Oliven grüne Oliven und/oder gereifte Oliven sind.

3. Verfahren nach Anspruch 1, bei dem die Oliven eingefrorene Oliven sind.

4. Verfahren nach Anspruch 1, bei dem man die zerkleinerten Oliven unter Unterdruck bei einer Temperatur unter oder gleich 80°C so trocknet, dass man getrocknete Oliven mit einem hohen Gehalt an wasserlöslichen Antioxidantien erhält, deren Gesamtwassergehalt 1-20 Gew.-% beträgt.

5. Verfahren nach Anspruch 1, bei dem die getrockneten Oliven einen Gesamtwassergehalt von 5-10 Gew.-% aufweisen.

6. Verfahren nach Anspruch 1, bei dem man an dem Kuchen mindestens eine Extraktion in einem Gewichtsverhältnis von MCT oder C₂-C₆-Alkylenglykol / Kuchen von 0,5 bis 2 vornimmt.

7. Verfahren nach Anspruch 1, bei dem das C₂-C₆₋Alkylenglykol Glykol, 1,2-Propylenglykol oder 1,3-Butylenglykol ist.

8. Fett- und wasserlösliche Oliven-Antioxidantien enthaltende Lipidfraktion der Pressung von ganzen getrockneten Oliven, deren Induktionszeit bei 110-120°C 15-75h beträgt und die nach dem Verfahren nach Anspruch 1 erhältlich ist.

9. Extrakt, der fett- und wasserlösliche Oliven-Antioxidantien, die Hydroxytyrosol, Tyrosol, Phenolsäuren und Oleuropein umfassen, enthält, der nach dem Verfahren nach einem der Ansprüche 1 bis 7 erhältlich ist.

10. Nahrungsmittel- oder kosmetische Zusammensetzung in Form einer Suspension oder einer Emulsion, die 0,5 - 4 Gew.-% des Extrakts, der mit fett- und wasserlöslichen Antioxidantien aus Oliven angereichert ist, gemäß Anspruch 9 enthält.

11. Nahrungsmittel- oder kosmetische Zusammensetzung in Form einer Suspension oder einer Emulsion, der eine Lipidfraktion enthält, die mit wasserlöslichen Antioxidantien gemäß Anspruch 8 angereichert ist.
